# EUROPÄISCHE PATENTANMELDUNG

(11) **EP 1 762 616 A1**
(43) Veröffentlichungstag der Anmeldung: **14.03.2007**
(21) Anmeldenummer: 05019914.0
(22) Anmeldetag: 13.09.2005
(51) Int. Cl.: C12N 15/10

(54) **Verfahren zur selektiven Anreicherung von doppelsträngiger DNA aus Nukleinsäuregemischen**

(71) Anmelder: QIAGEN GmbH, 40724 Hilden (DE); Forschungszentrum Jülich GmbH, 52425 Jülich (DE)
(72) Erfinder: Müller, Markus, 41542 Dormagen (DE); Kula, Maria-Regina, 81477 München (DE); Hubbuch, Jürgen, 50937 Köln (DE); Ferix, Andreas, 47546 Kalkar-Kehrum (DE)
(74) Vertreter: Leidescher, Thomas

(57) **Zusammenfassung**

Die Erfindung betrifft ein Verfahren zur Abreicherung unerwünschter Nukleinsäurebestandteile von doppelsträngiger DNA, insbesondere super-coiled Plasmid-DNA. Das erfindungsgemäße Verfahren ist gekennzeichnet durch die Schritte (a) Zurverfügungstellen eines Gemischs enthaltend vollständig und/oder teilweise doppelsträngige Nukleinsäuren sowie gegebenenfalls einzelsträngige Nukleinsäuren; (b) Resuspendieren des unter Punkt (a) genannten Gemischs in einem wäßrigen, niedermolaren Puffersystem mit niedriger Ionenstärke und geringer Pufferwirkung; (c) Einstellen von Bedingungen in dem Gemisch gemäß Schritt (b), bei welchen die vollständig und/oder teilweise doppelsträngigen Nukleinsäuren denaturiert werden; (d) weitere Zugabe von Puffer und einer Polymerkomponente zu dem Gemisch gemäß Schritt (c); (e) Inkubation des Gemischs gemäß Schritt (d) für einen Zeitraum, der ausreicht, ein wäßriges ZweiPhasensystem mit einer Ober- und einer Unterphase auszubilden; und (f) Entfernen der Einzelstrang-Nukleinsäure enthaltenden Oberphase und Auffangen der Doppelstrang-Nukleinsäure aus der Unterphase.

## Beschreibung

Die vorliegende Erfindung betrifft ein Verfahren zur selektiven Anreicherung von doppelsträngiger DNA, insbesondere von supercoil Plasmid-DNA, aus Nukleinsäuregemischen. Die vorliegende Erfindung befaßt sich mit der Abreicherung (Entfernung) von einzelsträngigen Nukleinsäuren, wie z.B. Ribonukleinsäure (RNA), denaturierter genomischer Desoxyribonukleinsäure (DNA) und/oder partiell denaturierter open circle Plasmid-DNA, aus Präparationen, die doppelsträngige Nukleinsäure, wie supercoil (sc) Plasmid-DNA, enthalten.

Im Stand der Technik finden sich zahlreiche Verfahren zur Isolierung von Plasmid-DNA sowohl im Kit- und "High-Throughput"- (HT) als auch im Produktions-Maßstab für therapeutische Anwendungen, wobei aber auf eine Abtrennung von (unter normalen Umständen doppelsträngig vorliegender) genomischer DNA (gDNA) und/oder open circle Plasmid-DNA (oc pDNA) von sc Plasmid-DNA (sc pDNA) in der Mehrzahl der Fälle nicht eingegangen wird.

Dennoch gibt es eine Reihe von Publikationen, die sich speziell mit dieser Thematik beschäftigen. In analytischen Anwendungen finden sich in erster Linie chromatographische Methoden zur oc/sc-Trennung von pDNA (z.B. TSKgel DEAE-NPR andere Produkte der TSKgel-Serie von Toso Biosciences), aber es werden auch kapillar-gelelektrophoretische Methoden und eine Reihe von molekularbiologischen Techniken über sequenz-spezifische Hybridisierungen (z.B. sog. *triple helix,* etc.) eingesetzt.

Für präparative Aufarbeitungen im Pilot- und Produktionsmaßstab von pDNA sind einige chromatographische Technologien beschrieben (PlasmidSelect-Harz von GE Healthcare und weitere HIC-Harze), welche allerdings durch aufwendige Handhabung, hohe Investitionskosten und vor allem durch geringe Ausbeuteeffizienzen gekennzeichnet sind.

In letzter Zeit finden sich auch Publikationen zum Einsatz von Zwei-Phasentrennungen zur präparativen Isolierung von Plasmid-DNA. In den nachfolgend erwähnten Arbeiten wurde allerdings das Problem der Abtrennung von einzelsträngiger DNA von doppelsträngiger DNA gar nicht oder technisch unbefriedigend beschrieben, vergl. z.B.

Kepka C, Rhodin J, Lemmens R, Tjerneld F, Gustavsson P-E., 2004, Extraction of plasmid DNA from Escherichia coli cell lysate in a thermoseparating aqueous two-phase system*1, Journal of Chromatography A 1024(1-2):95-104.; Frerix, A., Müller, M., Kula. M.-R., and Hubbuch J. Scalable recovery of plasmid DNA based on aqueous two-phase separation, Biotechnol. Appl. Biochem. (2005) 042, 37-66; Ribeiro S.C., Monteiro G.A., Cabral J.M.S., Prazeres D.M.F., 2002, Isolation of plasmid DNA from cell lysates by aqueous two-phase systems, Biotechnology and Bioengineering 78(4):376-384.

Z.B. sind die beschriebenen Verfahren zur oc/sc-Trennung, wie die Hydrophobe Interaktions Chromatographie (HIC) und die Thiophile Chromatographie (Lemmens R., Olsson U., Nyhammar T., Stadler J., 2003, Supercoiled plasmid DNA: selective purification by thiophilic/aromatic adsorption, Journal of Chromatography B-Analytical Technologies in the Biomedical and Life Sciences 784(2):291-300) und die Gegenstrom-Chromatographie (Kendall D., Booth A.J., Levy M.S., Lye G.J., 2001, Separation of supercoiled and opencircular plasmid DNA by liquid-liquid counter-current chromatography, Biotechnology Letters 23(8):613-619) sehr zeitaufwendig, aufgrund niedriger Kapazitäten und/oder mangelnder Ausbeuten sehr teuer, was als nachteilig an diesen Verfahren anzusehen ist.

Aufgabe der vorliegenden Erfindung ist es daher, ein Verfahren anzugeben, bei dem eine Abtrennung von selektiv (partiell) denaturierter gDNA und oc pDNA sowie anderen einzelsträngigen Nukleinsäuren von doppelsträngiger/doppelsträngigen Nukleinsäure(n), wie sc Plasmid-DNA, ermöglicht wird, ohne die oben genannten Nachteile der bekannten Verfahren in Kauf nehmen zu müssen. Speziell ist die Aufgabe darin zu sehen, eine Abtrennung von sc pDNA von anderen Nukleinsäuren zu ermöglichen.

Diese Aufgabe löst die Erfindung durch das im unabhängigen Anspruch 1 angegebene Verfahren. Weitere vorteilhafte Ausgestaltungen des erfindungsgemäßen Verfahrens ergeben sich aus den abhängigen Ansprüchen, der Beschreibung, den Beispielen und der Zeichnung.

Die Erfindung betrifft ein Verfahren zur gezielten Abreicherung von einzelsträngigen Nukleinsäuren, wie z.B. RNA, denaturierter genomischer DNA und partiell denaturierter open circle Plasmid-DNA, aus Präparationen, die ebenfalls doppelsträngige Nukleinsäuren, wie supercoil Plasmid-DNA, enthalten. In einem speziellen Fall ermöglicht die vorliegende Erfindung z.B. die selektive Denaturierung von gDNA und oc pDNA sowie deren anschließende Abtrennung durch Extraktion in einem Zweiphasensystem. Es handelt sich somit um eine Feinreinigung hin zu doppelsträngigen Nukleinsäuren, wie z.B. sc pDNA. Das Verfahren zeichnet sich dadurch aus, daß Anteile an doppelsträngiger Nukleinsäuren, wie genomischer DNA, loop-bildender RNA und nativer, doppelsträngiger oc-pDNA, durch Denaturierung vollständig oder partiell selektiv in Einzelstränge überführt und anschließend in einem wäßrigen Zwei-Phasensystem selektiv von doppelsträngiger Nukleinsäure, wie z.B. sc Plasmid-DNA, mit hoher Effizienz und Kapazität getrennt werden können. Der Denaturierungsschritt kann vorzugsweise durch stark alkalische Bedingungen (z.B. Zugabe von NaOH, KOH etc.) oder Hitze-Inkubation (z.B. Erwärmung auf ≥70 °C, insbesondere ≥80 °C, abhängig vom GC-Gehalt der Nukleinsäure) induziert werden. Im Vergleich zu existierenden konventionellen (z.B. chromatographischen) Methoden liegen die Vorteile der vorgestellten Erfindung besonders in den deutlich geringeren Kosten, der signifikant schnelleren Durchführbarkeit und der einfacheren Automatisierbarkeit der Methodik.

Die vorliegende Erfindung betrifft ein Verfahren zur selektiven Abreicherung von teilweise und vollständig denaturierten Nukleinsäuren von doppelsträngigen Nukleinsäuren, insbesondere sc pDNA. Das Verfahren ist besonders geeignet zur Herstellung von sc pDNA Präparationen im Pilot- und Produktionsmaßstab, z.B. zur Herstellung sc Plasmid-DNA zur humanen genetischen Vakzinierung oder für gentherapeutische Anwendungen, eignet sich aber aufgrund seiner Einfachheit auch zum Einsatz in manuellen Kit- und automatisierten high-throughput (HT) Applikationen, z.B. in der Diagnostik. Das erfindungsgemäße Verfahren eignet sich besonders gut für die selektive Abreicherung von einzelsträngigen Nukleinsäuren und open circle (oc) Plasmid-DNA aus supercoil Plasmid-DNA enthaltenden Präparationen.

Bei der Aufreinigung von Plasmidmolekülen zur klinischen oder diagnostischen Anwendung stehen zum einen die zu erreichende Produktqualität und zum anderen die im Verhältnis dazu entstehenden Aufarbeitungskosten (*cost-of-goods,* COGs) im Focus der Prozessentwicklung. Hierbei kann sich die Zielsetzung hinsichtlich der benötigten Reinheit des Zielmoleküls (z.B. pDNA) deutlich unterscheiden. So sind die gewünschten und benötigten Reinheitsgrade bei klinischen Anwendungen deutlich höher als sie im Vergleich z..B. bei den meisten diagnostischen Anwendungen vonnöten sind. In beiden Gebieten allerdings ist es das Ziel von Verfahrensoptimierungen, die Kosten der Aufreinigung auf ein Minimum zu reduzieren, um kommerzielle Anwendungen zu ermöglichen. Dieses Ziel kann nur durch die Entwicklung hoch-auflösender Reinigungmethoden erreicht werden, da hierdurch gleichzeitig die Anzahl der benötigten Aufreinigungsschritte so gering wie möglich gehalten werden kann.

Diese Zielsetzung (d.h. eine möglichst effiziente Reinigung von Plasmid-DNA) wird umso schwieriger, je mehr die abzureichernden Kontaminanten physiko-chemisch dem Zielmolekül ähnlich sind. So unterscheidet sich z.B. sogenannte open circle (oc) Plasmid-DNA von supercoil (sc) Plasmid-DNA im wesentlichen nur durch einen Strangbruch oder mehrere Strangbrüche in einem Strang oder beiden Strängen der doppelhelicalen Struktur des Plasmidmoleküls, was in der Konsequenz auch zur sterischen Unterschieden zwischen beiden topologischen Formen führt. Das Entstehen von oc pDNA aus sc pDNA erfolgt vorwiegend durch enzymatisches oder mechanisches nicking der in vivo primär vorliegenden sc pDNA. Dabei entsteht eine sc pDNA, wenn vor dem Schließen von zwei Einzelsträngen zu einem Doppelstrang ein einzelner der beiden Stränge oder beide Stränge gedreht werden, so daß nach dem Schließen zum Doppelstrang durch die entstehenden Spannungen Schlaufen ("supercoils") ausgebildet werden.

Die vorliegende Erfindung ermöglicht es erstmals, sowohl hoch-selektiv wie auch äußerst einfach, schnell und insbesondere kostengünstig die gewünschte Trennung von Nukleinsäuren zu erreichen. Dabei wird nach einer schonenden Behandlung zur Gewinnung einzel- und doppelsträngiger Nukleinsäuren in einer nachgeschalteten Zwei-Phasen-Trennung, wie in der WO2004/106516 A1 beschrieben, eine nahezu quantitative Separation von (partiell) einzelsträngiger DNA (z.B. denaturierter oc pDNA) von doppelsträngiger DNA (z.B. sc pDNA) erzielt. Dies wird durch kostengünstige Einsatzstoffe, die unproblematisch zu entsorgen sind, bei gleichzeitiger hoher spezifischer Kapazität der beschriebenen Erfindung erreicht.

Die vorliegende Erfindung beschreibt somit die gezielte vollständige oder partielle Denaturierung von doppelsträngigen Nukleinsäuren, beispielsweise durch Einwirkung von alkalischen pH-Werten von 11 oder höher, oder durch Hitze. Eine solche Denaturierung hat zur Folge, daß einzelsträngige Nukleinsäuren, wie DNA oder RNA, aufgrund veränderter Lösungseigenschaften im Vergleich zu doppelsträngigen Nukleinsäuren, wie DNA, in Phasensystemen anderen Verteilungskoeffizienten folgen.

Im Unterschied zu z.B. oc pDNA denaturiert sc pDNA auch während der Denaturierungsphase, renaturiert aber dennoch aufgrund der dreidimensionalen Topologie und der daraus resultierenden sterischen Stabilisierung des sog. supercoils z.B. nach Neutralisation oder Abkühlung vollständig zurück in die supercoil-Doppelhelix-Struktur. Verglichen mit doppelsträngiger DNA weisen einzelsträngige DNA und RNA eine hydrophobere Oberfläche auf, die auf das Vorhandensein freiliegender Basen zurückzuführen ist. Aufgrund der unterschiedlichen Re- und Denaturierungseigenschaften und der daraus resultierenden Struktureigenschaften, Hydrophobizitäten und Ladungsdichten von z.B. oc und sc pDNA kann eine hoch-selektive Separation beider Plasmid-Topoisomere durch Extraktion im wässrigen Zwei- Phasensystemen erreicht werden.

Dieser Mechanismus wird bei der vorliegenden Erfindung bewußt eingesetzt, um die normalerweise äußerst geringen Unterschiede der Oberflächeneigenschaften von sc pDNA und oc pDNA sowie gDNA durch absichtliche selektive Denaturierung erheblich zu verstärken, wodurch eine spätere Abtrennung hocheffizient ermöglicht wird.

Für das erfindungsgemäße Verfahren wird in Schritt (d) ein Puffer zugesetzt. Vorzugsweise wird hier ein Kaliumphosphatpuffer verwendet. Dabei enthält der Puffer besonders bevorzugt ein Gemisch aus K₂HPO₄ und KH₂PO₄. Die erfindungsgemäßen Puffer werden vorzugsweise mit einem pH-Wert im Bereich von pH 5,8 bis pH 8,5 und insbesondere bevorzugt mit einem pH Wert im Bereich von pH 6,5 bis pH 8 eingesetzt. Beispielsweise ist eine Zusammensetzung der Stammlösung von 3,83 M K₂HPO₄ und 2,45 M KH₂PO₄ und einer PEG 800-Konzentration von (es ergibt sich ein pH-Wert von ca. 7) im erfindungsgemäßen Verfahren besonders bevorzugt anwendbar. Dabei werden K₂HPO₄ und KH₂PO₄ bezogen auf das Zwei-Phasensystem z.B. in einer Gesamtkonzentration von 5 - 30 % (w/w) eingesetzt, vorzugsweise in einer Gesamt-Konzentration von 10 - 25 % (w/w) und besonders bevorzugt in einer Gesamt-Konzentration von 20 % (w/w). Die Zugabe des Kaliumphosphats erfolgt üblicherweise in einem Temperaturbereich zwischen eisgekühlt und Raumtemperatur. Raumtemperatur im Sinne der vorliegenden Erfindung bezeichnet einen Temperaturbereich von 18 bis 25°C. Vorzugsweise wird im erfindungsgemäßen Verfahren ein eisgekühlter Phosphatpuffer eingesetzt. Eine Inkubation ist vorteilhafterweise nach der Zugabe des Kaliumphosphats nicht erforderlich, entscheidend ist eine möglichst vollständige und gleichmäßige Durchmischung der Lösung nach der Puffer-Zugabe. Wenn doch inkubiert wird, beträgt die Inkubationsdauer üblicherweise etwa 1 bis 15 Minuten. Vorzugsweise wird der Ansatz, wie oben erwähnt, während und/oder nach der Zugabe der Salz-Komponente bewegt, wie z.B. stark geschüttelt, gerührt oder ähnliches.

Bei der Polymer-Komponente, die gemäß der Erfindung eingesetzt wird, handelt es sich vorzugsweise um Polyethylenglycol (PEG). Das Polyethylenglykol wird vorzugsweise mit einem Molekulargewicht von im rechnerischen Mittel 600 bis 1000 g/mol, mehr bevorzugt von im rechnerischen Mittel 700 - 900 g/mol und besonders bevorzugt von im rechnerischen Mittel 750 - 880 g/mol, als eine der beiden Komponenten des Zwei-Phasensystems eingesetzt. Das bei der vorliegenden Erfindung verwendete PEG besteht vorzugsweise aus einem Gemisch aus Polyethylenglykol mit einem durchschnittlichen Molekulargewicht von 600 g/mol (PEG 600) und Polyethylenglykol mit einem durchschnittlichen Molekulargewicht von 1000 g/mol (PEG 1000). Beide Polyethylenglycole sind kommerziell erhältlich (z.B. Fluka, Buchs, Schweiz). Das einsatzfertige PEG-Gemisch besteht dabei z.B. aus 30 - 50 % (w/w) PEG 600 und 50 - 70 % (w/w) PEG 1000, vorzugsweise 33 - 45 % (w/w) PEG 600 und 55 - 67 % (w/w) PEG 1000, besonders bevorzugt aus 36 - 40 % (w/w) PEG 600 und 60 - 64 % (w/w) PEG 1000 und ganz besonders bevorzugt aus 38 % (w/w) PEG 600 und 62 % (w/w) PEG 1000.

Die Konzentration des PEG im erfindungsgemäßen, wäßrigen Zwei-Phasensystem wird so gewählt, daß sich mit der Salz-Komponente zwei Phasen ausbilden, wobei jedoch die PEG-Konzentration, bei der die doppelsträngige DNA, z.B. Plasmid-DNA, aus der Unterphase, in der sie sich bei niedrigeren Konzentrationen befindet, in die Oberphase wechselt, nicht überschritten wird. Vorzugsweise beträgt der PEG-Gehalt in dem Gesamtgemisch mindestens 10 % (w/w) und wird nach oben begrenzt durch die Konzentration von PEG, bei der die doppelsträngige DNA (z.B. Plasmid-DNA) aus der Unterphase, in der sie sich bei niedrigeren Konzentrationen befindet, in die Oberphase wechselt. Nach der Zugabe von PEG sollte die Lösung vorzugsweise eine Temperatur von etwa 10 bis 50°C aufweisen, besonders bevorzugt eine Temperatur von etwa 15 bis 40°C. Die doppelsträngige DNA (z.B. Plasmid-DNA) findet sich nach Ausbildung der Phasen, was je nach Volumen des Ansatzes einige Minuten bis Stunden in Anspruch nehmen kann, in der salzhaltigen Unterphase. Die Ausbildung der Phasen kann optional durch Zentrifugation des Ansatzes beschleunigt werden, wodurch es vorteilhafterweise zu einer weiteren zeitlichen Verkürzung des erfindungsgemäßen Verfahrens kommt. Die Bedingungen, unter welchen man einen solchen Zentrifugationsschritt durchführt, sind dem Fachmann geläufig.

Wäßrige Zwei-Phasensysteme haben gegenüber festen adsorptiven Phasen den Vorteil, daß sie eine weitaus höhere Kapazität für die aufzureinigende doppelsträngige DNA (wie Plasmid-DNA) haben, welche praktisch nur durch die Löslichkeit in den Phasen begrenzt ist. Weiterhin ist das Verfahren aufgrund der benötigten sehr einfachen Apparaturen nahezu beliebig dimensionierbar. Aber erst mit den hier beschriebenen Vereinfachungen können sowohl eine Automatisierung als auch unabhängig davon eine Produktion im industriellen Maßstab, beispielsweise zur Produktion von >> 2 g hochgereinigter Plasmid-DNA pro Ansatz, leicht erreicht werden. Ebenso kann mit der vorliegenden Erfindung vorteilhafterweise die doppelsträngige DNA (wie Plasmid-DNA) in sehr hohem Maße von RNA und denaturierter gDNA befreit werden, was in vielen Anwendungen, besonders in klinischen, z.T. regulatorisch gefordert ist . Plasmid-DNA, welche nach dem erfindungsgemäßen Verfahren feingereingt ("Polish") wird, ist besonders nach einem primären Reinigungsschritt (beispielsweise über QIAGEN-Resin, QIAGEN, Hilden, Deutschland) innerhalb der in der Gentherapie oder der genetischen Vakzinierung gegenwärtig akzeptierten Freigabespezifikationen. So können vorteilhafterweise große Mengen hochreiner Plasmid-DNA mit sehr geringem apparativen Aufwand unter Verwendung nicht-toxischer Substanzen und bei Anfall vergleichsweise geringer Kosten produziert werden. Hierzu sei erwähnt, daß die im erfindungsgemäßen Zwei-Phasensystem eingesetzten Substanzen im Vergleich zu anderen Isolierungsmethoden, wie z.B. CsCl-Dichtegradienten-Zentrifugation oder Phenolextraktion, ökologisch unbedenklich sind und vollständig und leicht aus der aufgereinigten Plasmid-DNA entfernt werden können.

Bei dem erfindungsgemäßen Verfahren wird die die doppelsträngige DNA enthaltende, sich in Schritt (e) einstellende Unterphase von der die unerwünschten Nukleinsäuren enthaltenden Oberphase getrennt, woraufhin die gewünschte doppelsträngige DNA in angereicherter Form aus der Unterphase gewonnen werden kann (Schritt (f)). Obwohl mit einer einmaligen Phasentrennung bereits ein hoher Abreicherungsgrad erzielt werden kann, kann die Effizienz des erfindungsgemäßen Verfahrens noch gesteigert werden, indem die Schritte (d) bis (f) ein bis mehrmals wiederholt oder die Extraktionen im Gegenstromprinzip durchgeführt werden. Sinnvoll ist z.B. eine ein- bis dreimalige Wiederholung der Verfahrenschritte (d), (e) und (f). Für höchstangereicherte, doppelsträngige Nukleinsäure(n), wie sc Plasmid-DNA, können die Schritte (d) bis (f) auch mehr als dreimal wiederholt werden, bis die gewünschte Reinheit erreicht ist. Die doppelsträngige DNA (z.B. Plasmid-DNA) findet sich jeweils in der Unterphase. Die Durchführung dieses optionalen Schrittes führt zu einer wiederholten Reinigung der doppelsträngigen DNA (z.B. Plasmid-DNA) und damit zu einer weiteren Abreicherung von Kontaminanten, wie z.B. RNA, aus der doppelsträngigen DNA (z.B. Plasmid-DNA).

Im Anschluß an das erfindungsgemäße Verfahren ist es sinnvoll, die in der Unterphase befindliche doppelsträngige DNA (wie sc Plasmid-DNA) zu isolieren. Die Isolierung und Entsalzung der (Plasmid)-DNA aus der in Schritt e) entstehenden Unterphase kann z.B. durch Ultrafiltration, Diafiltration oder Gelfiltration erfolgen. Es kann jedoch im Sinne der vorliegenden Erfindung auch jede andere dem Fachmann bekannte Methode zur Isolierung und/oder Entsalzung der (Plasmid)-DNA aus der Unterphase verwendet werden.

Der in Schritt (b) zugesetzte wäßrige, niedermolare Puffer ist vorzugsweise ein schwacher Puffer, der nur eine niedrige Ionenstärke aufweist. Die Molarität des eingesetzten Puffers beträgt vorzugsweise nicht mehr als 100 mM, mehr bevorzugt nicht mehr als 50 mM, und insbesondere nicht mehr als 10 mM.

Geeignete wäßrige, niedermolare Puffersysteme sind z.B. ein Trispuffer, ein Tris/EDTA-Puffer, eine phosphatgepufferte Salzlösung (PBS) oder ein Citratpuffer wie auch andere dem Fachmann geeignet erscheinende Puffersysteme.

Für die Einstellung denaturierender Bedingungen in Schritt (c) bieten sich einerseits die Erhöhung des pH-Werts der Lösung auf 11 oder darüber oder eine Temperaturerhöhung auf 70°C oder höher an. Die Erhöhung des pH-Werts kann in bekannter Weise durch Zugabe einer starken Base, wie NaOH oder KOH, erfolgen. Bei einer Temperaturerhöhung ist es von Vorteil, wenn die gewählte Temperatur zwischen etwa 70 und 95°C, insbesondere etwa 80 und 95°C, liegt Die optimale Temperatur hängt dabei vom GC-Gehalt der vorhandenen Nukleinsäuren ab.

Als besonders vorteilhaft hat es sich erwiesen, wenn das erfindungsgemäße Verfahren im Anschluß an eine Vorreinigung bzw. Vortrennung durchgeführt wird, wobei jedes bekannte Aufreinigungsverfahren für die Vorreinigung/Vortrennung verwendet werden kann. Eine besonders geeignete Vorreinigung/Vortrennung ist z.B. eine wäßrige Zweiphasentrennung, wie sie aus der WO 2004/106516 A1 bekannt ist. Bei entsprechendem Vorgehen kann ein sehr hoher Reinigungsgrad erzielt werden. Es ist so beispielsweise möglich, den Gehalt an sc pDNA in einer Probe, bezogen auf die vorhandene gesamte Nukleinsäuremenge, insgesamt auf 90 % und mehr, insbesondere auf 95 % und mehr, und sogar auf 99 % und mehr zu steigern. Ein anders besonders geeignetes Verfahren für die Vorreinigung/Vortrennung ist die Anionenaustauscherchromatographie, bei der vergleichbare prozentuale sc pDNA-Gehalte in einer Probe erreicht werden können.

Die vorliegende Erfindung wird nachfolgend anhand von Beispielen näher erläutert.

### Beispiel 1

Dieses Beispiel befaßt sich mit der Denaturierung von oc pDNA unter alkalischen Bedingungen und Abreicherung im Zwei-Phasen-System.

Eine vorgereinigte und aufkonzentrierte Plasmid-DNA-Präparation enthaltend oc pDNA und sc pDNA sowie die weitere Nukleinsäuren RNA und partiell doppelsträngige gDNA wird bei stark alkalischem pH-Wert (> 11) inkubiert. Unter diesen Bedingungen wird eine Denaturierung (Strangtrennung) der DNA-Doppelhelix bzw. der Doppelstrang-RNA (z.B. tRNAs) erreicht, die nur bei intakter supercoil-DNA in einem Grenzbereich reversibel ist. Nach Überführung des Denaturierungsansatzes in ein gepuffertes Phasensystem, welches auf die Trennung von oc/sc pDNA Topoisomeren optimiert ist, erfolgt eine effiziente und hochauflösende Abtrennung der oc pDNA, gDNA sowie der partiell doppelsträngigen RNA von dem Zielmolekül sc pDNA.

Die Durchführung war wie folgt. Es wurden 15 g einer Plasmid-haltigen Ausgangslösung (36 µg/ml, bestimmt mittels HPLC) mit 5 g NaOH (0.4 M NaOH) versetzt. Der Reaktionsansatz wurde gemischt und für 5 min bei Raumtemperatur inkubiert. Danach wurden 20 g Kaliumphosphatpuffer (50 % w/w, pH 7.4) sowie 10 g PEG 800 (75 % w/w) zugegeben. Diese Zusammensetzung wurde wiederum gut gemischt. Nach Vermischen trat eine typische Trübung des Ansatzes ein. Durch Zentrifugation kann das Absetzen des Ober- und Unterphase beschleunigt werden (z.B. 5 min bei 2000g). Die Abgetrennte Unterphase enthält die gereinigte sc pDNA, während denaturierte DNA (oc pDNA und gDNA) als weisser "Schmier" in der Phasengrenze (Interphase, zwischen Unter- und Oberphase) sichtbar ist.

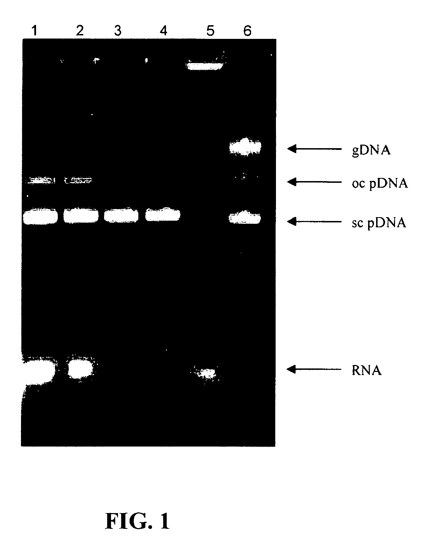

Die Ergebnisse sind in dem in Fig. 1 abgebildeten 0,8 % Agarosegel zu sehen. Es zeigen:

| Spuren | Probe |
|---|---|
| 1+2 | Plasmidhaltige Ausgangslösung |
| 3+4 | Plasmid in entsalzter Unterphase nach alkalischer Denaturierung und Zweiphasen- Extraktion |
| 5 | Interphase aus der wäßrigen Zwei-Phasentrennung, in TE (10mM Tris/Cl, 1mM EDTA, pH 8.0) resuspendiert |
| 6 | gDNA, pDNA Standard |

Die Proben 1 und 2 bzw. 3 und 4 sind vor bzw. nach der Reinigung in identischen Volumenverhältnissen doppelt aufgetragen worden. Es ist aus dem in Fig. 1 gezeigten Agarosegel klar erkennbar, daß von den Nukleinsäuren in den Spuren 3 und 4, die einem erfindungsgemäßen Verfahren unterzogen und aus der Unterphase nach der wäßrigen Zwei-Phasentrennung entnommen wurden, der Anteil an oc pDNA (dritte Bande von unten) stark vermindert ist im Vergleich zu den Spuren 1 und 2, welche die Ausgangslösung repräsentieren. Das Zielmolekül sc pDNA (zweite Bande von unten) liegt in hochgereinigter Form vor. Auch ist aus den Spuren 3 und 4 von Fig. 1 ersichtlich, daß durch die erfindungsgemäße Behandlung der Nukleinsäureprobe die niedermolekularen RNA-Reste (unterste Bande) der Probe praktisch quantitativ entfernt wurden. In Spur 5 (Interphase) schließlich sind nur die niedermolekularen RNA-Reste (unterste Bande) sowie denaturierte DNA als Taschenkontamination erkennbar.

### Beispiel 2

Dieses Beispiel befaßt sich mit der Denaturierung von oc pDNA durch Hitze-Inkubation und Abreicherung im Zwei-Phasen-System. Es wurden insgesamt 350 µl enthaltend pDNA (100 µg/ml) und gDNA (49 µg/ml) in TE vorgelegt. Anschließend wurde der Ansatz auf Temperaturen von 70 bis 95°C (in 5°C-Schritten) erwärmt, jeweils für 5 min inkubiert und anschließend jeweils für 5 min auf Eis abgekühlt. Dann wurden 300 mg der Proben mit 300 mg TE vermischt, 600 mg Phosphatpuffer (50 %, w/w) und 300 mg PEG (75 %, w/w) zugegeben und vermischt. Daraufhin wurde der Ansatz zentrifugiert. Das Gesamtvolumen war 1,2 ml, davon entfielen 650 µl auf die Unterphase, welche die gereinigte sc DNA enthielt.

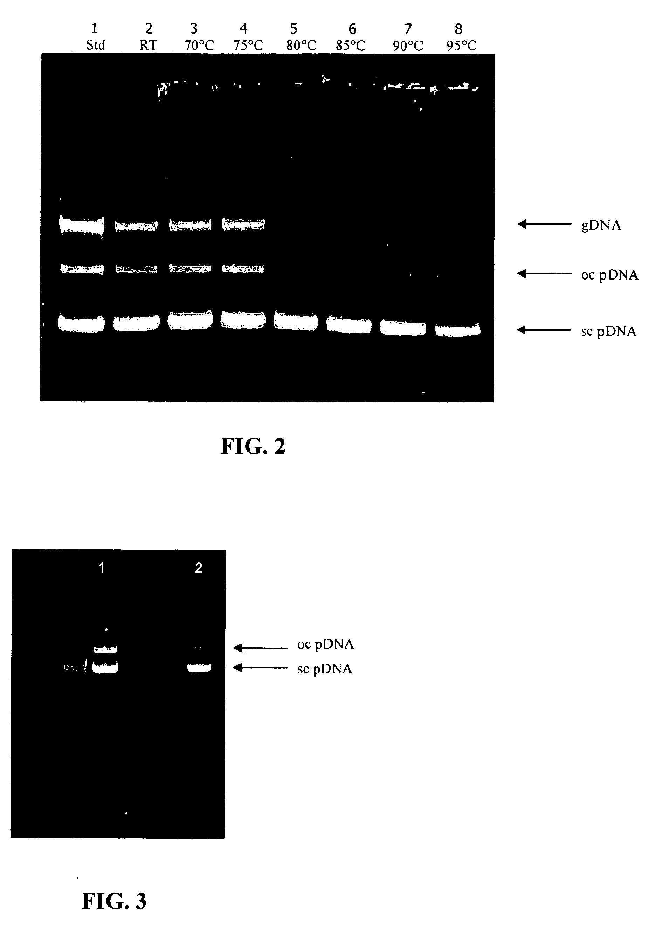

Die Ergebnisse sind in dem in Fig. 2 abgebildeten Agarosegel 0,8 % zu sehen. Es zeigen:

| Spur | Probe |
|---|---|
| 1 | pDNA und gDNA bei RT (Standard (Std), entsprechend 100 % Ausbeute) |
| 2 | pDNA und gDNA bei RT, anschließend wäßriges Zwei-Phasensystem |
| 3 | 5 min bei 70°C; anschließend auf Eis und wäßriges Zwei-Phasensystem |
| 4 | 5 min bei 75°C; anschließend auf Eis und wäßriges Zwei-Phasensystem |
| 5 | 5 min bei 80°C; anschließend auf Eis und wäßriges Zwei-Phasensystem |
| 6 | 5 min bei 85°C; anschließend auf Eis und wäßriges Zwei-Phasensystem |
| 7 | 5 min bei 90°C; anschließend auf Eis und wäßriges Zwei-Phasensystem |
| 8 | 5 min bei 95°C; anschließend auf Eis und wäßriges Zwei-Phasensystem |

Die unterste Bande des in Fig. 2 gezeigten Agarosegels zeigt sc PDNA. Es ist des weiteren aus dem Gel ersichtlich, daß unter den gegebenen Bedingungen und für das eingesetzte Plasmid pCMVβ eine Denaurieungstemperatur von 80°C bei 5 min Inkubation ausreichend ist, um eine praktisch quantitative Abtrennung von gDNA und oc pDNA von sc pDNA zu erreichen.

### Beispiel 3

Dieses Beispiel betrifft die oc pDNA-Abreicherung ("polishing") eines Vakzinierungsplasmides mittels alkalischer Denaturierung und eines wäßrigen Zwei-Phasensystems nach einer Primäraufreinigung durch Anionenaustauscherchromatographie.

Die Herstellung von Plasmid-DNA für gentherapeutische und genetische Vakzinierungen unterliegt strengen Regularien und hohen Spezifikationen. Gewisse Plasmidsequenzen neigen dazu, in der Aufarbeitung "genickt" zu werden, d.h. Einzelstrangbrüche zu erhalten, und somit hohe Anteile an oc pDNA auszubilden. Die entstandenen Anteile an oc pDNA müssen nach einer ersten Aufreinigung abgereichert werden. Im vorliegenden Fall erfolgte diese Abreicherung über Anionenaustauschchromatographie.

Die Versuchsdurchführung war wie folgt. Es wurden 59,24 g einer Plasmidlösung (4 mg/ml) in 540,8 g einesTris/EDTA-Puffers (pH-Wert 8) gegeben. Anschließend wurden 200 g NaOH (0.4 M) zugegeben und gemischt. Das Gemisch wurde dann für 5 Minuten bei Raumtemperatur inkubiert. Dem Gemisch wurden anschließend 700 g Kaliumphosphatpuffer (50% w/w, pH 7.4) sowie 400 g PEG 800(75 % w/w, 60°C) zugefügt. Es wurde wiederum gemischt und bei 3000 x g für 10 min abzentrifugiert. Die Unterphase (ca. 900 ml) enthält gereinigte sc pDNA, welche über z.B. Ultrafiltration oder Gelfiltration in geeignete Formulierungslösungen überführt wird.

Die Ergebnisse sind in dem in Fig. 3 abgebildeten Agarosegel (0,8 %) zu sehen. Es zeigen:

| | | | |
|---|---|---|---|
| Spur 1 | (Vor "polish"): | Gesamt pDNA: 236,6 mg; | |
| | Tasche: | 4,1 % hauptsächlich gDNA; | |
| | oc: | 28,4 % | (absolut: 67,2 mg oc pDNA); |
| | sc: | 67,5 % | (absolut: 159,7 mg sc pDNA); |

| | | | |
|---|---|---|---|
| Spur 2 | (Nach "polish") | Gesamt pDNA: 168 mg | |
| | Tasche: | Keine detektierbaren Signale; | |
| | oc: | 14,9% | (absolut: 25 mg oc pDNA) |
| | sc: | 85,1% | (absolut: 142 mg sc pDNA) |

Wie ein Vergleich der beiden Spuren 1 und 2 des in Fig. 3 gezeigten Gels zeigt, ist die oc pDNA-Bande nach dem Reinigungsschritt (nach "polish", siehe Spur 2) erheblich schwächer ausgebildet aus vor dem Reinigungsschritt (vor "polish", siehe Spur 2). Die quantitativen Ergebnisse dazu sind der obigen Tabelle zu entnehmen.

Wie sich aus den obigen Ausführungen entnehmen läßt, kann durch das erfindungsgemäße Verfahren in einer biologischen Probe die Menge an oc Plasmid-DNA von über 67 mg auf 25 mg reduziert werden, d.h. eine Reduktion um etwa 62 % erzielt werden. Dagegen wurden von den 159,7 mg sc pDNA in der Ausgangsprobe nach Durchführung des erfindungsgemäßen Verfahrens noch 142 mg wieder gefunden, was einer Ausbeute von etwa 89 % entspricht.

## Patentansprüche

1. Verfahren zum Entfernen einzelsträngiger Nukleinsäuren von doppelsträngigen Nukleinsäuren, **gekennzeichnet durch** die folgenden Schritte:
(a) Zurverfügungstellen eines Gemischs enthaltend vollständig und/oder teilweise doppelsträngige Nukleinsäuren sowie gegebenenfalls einzelsträngige Nukleinsäuren;
(b) Resuspendieren des unter Punkt (a) genannten Gemischs in einem wäßrigen, niedermolaren Puffersystem mit niedriger Ionenstärke und geringer Pufferwirkung;
(c) Einstellen von Bedingungen in dem Gemisch gemäß Schritt (b), die zu einer reversiblen Denaturierung von einer bestimmten doppelsträngigen Nukleinsäure oder mehrerer bestimmter doppelsträngiger Nukleinsäuren führen, wobei eine andere Nukleinsäure oder mehrere andere Nukleinsäuren irreversibel denaturiert werden;
(d) weitere Zugabe von Puffer und einer Polymerkomponente zu dem Gemisch gemäß Schritt (c);
(e) Inkubation des Gemischs gemäß Schritt (d) für einen Zeitraum, der ausreicht, ein wäßriges Zwei-Phasensystem mit einer Ober- und einer Unterphase auszubilden; und
(f) Entfernen der Einzelstrang-Nukleinsäure enthaltenden Ober- sowie Interphase und Auffangen der Doppelstrang-Nukleinsäure aus der Unterphase.

2. Verfahren nach Anspruch 1, **dadurch gekennzeichnet, daß** das Gemisch aus Schritt (a) supercoil (sc) Plasmid-DNA enthält.

3. Verfahren nach Anspruch 1 oder 2, **dadurch gekennzeichnet, daß** die bestimmte doppelsträngige Nukleinsäure aus Schritt (c), die reversibel denaturierbar ist, supercoil (sc) Plasmid-DNA ist.

4. Verfahren nach einem der Ansprüche 1 bis 3, **dadurch gekennzeichnet, daß** das Gemisch aus Schritt (a) open circle (oc) Plasmid-DNA enthält.

5. Verfahren nach einem der Ansprüche 1 bis 4, **dadurch gekennzeichnet, daß** in Schritt (f) einzelsträngige oc Plasmid-DNA in der Oberphase von doppelsträngiger sc Plasmid-DNA in der Unterphase voneinander getrennt werden.

6. Verfahren nach einem der vorhergehenden Ansprüche, **dadurch gekennzeichnet, daß** das wäßrige, niedermolare Puffersystem mit niedriger Ionenstärke und geringer Pufferwirkung gemäß Schritt (b) eine Molarität von bis zu 100 mM aufweist.

7. Verfahren nach einem der vorhergehenden Ansprüche, **dadurch gekennzeichnet, daß** das wäßrige, niedermolare Puffersystem gemäß Schritt (b) ein Trispuffer, ein Tris/EDTA-Puffer, phosphatgepufferte Salzlösung (PBS, phosphate buffered saline), oder ein Citratpuffer ist.

8. Verfahren nach einem der vorhergehenden Ansprüche, **dadurch gekennzeichnet, daß** die denaturierenden Bedingungen gemäß Schritt (c) durch eine Erhöhung des pH-Werts auf 11 oder höher mit anschliessender ausreichender Inkubation hergestellt werden.

9. Verfahren nach einem der vorhergehenden Ansprüche, **dadurch gekennzeichnet, daß** die denaturierenden Bedingungen gemäß Schritt (c) durch eine Erhöhung der Temperatur auf 70°C oder höher hergestellt werden und nach Abschluss einer Inkubation eine sofortige Abkühlung erfolgt.

10. Verfahren nach einem der vorhergehenden Ansprüche, **dadurch gekennzeichnet, daß** der gemäß Schritt (d) zugegebene weitere Puffer ein Kaliumphosphatpuffer ist.

11. Verfahren nach einem der vorhergehenden Ansprüche, **dadurch gekennzeichnet, daß** das gemäß Schritt (d) zugegebene Polymer ein Polyethylenglykol (PEG), vorzugsweise ein PEG mit einem Molekulargewicht von 600 bis 1000 g/mol, mehr bevorzugt von 700 bis 900 g/mol, und insbesondere von 750 bis 880 g/mol, jeweils bezogen auf das rechnerische Mittel, ist

12. Verfahren nach einem der vorhergehenden Ansprüche, **dadurch gekennzeichnet, daß** die angereicherte doppelsträngige Nukleinsäure aus der Unterphase durch Ultrafiltration oder Gelfiltration weiter konzentriert wird.

13. Verfahren nach Anspruch 12, **dadurch gekennzeichnet, daß** es sich bei der in der Unterphase angereicherten doppelsträngigen Nukleinsäure um sc Plasmid-DNA handelt.

14. Verfahren nach einem der vorhergehenden Ansprüche, **dadurch gekennzeichnet, daß** die Schritte (d), (e) und (f) mindestens einmal wiederholt werden.

15. Verfahren nach Anspruch 14, **dadurch gekennzeichnet, daß** die Schritte (d), (e) und (f) dreimal wiederholt werden.

16. Verfahren nach einem der vorhergehenden Ansprüche, **dadurch gekennzeichnet, daß** nur die Unterphase in Schritt (e) sc Plasmid-DNA enthält.

17. Verfahren nach einem der vorhergehenden Ansprüche, **dadurch gekennzeichnet, daß** das Verfahren im Anschluß an eine Vortrennung/Vorreinigung durchgeführt wird.

18. Verfahren nach Anspruch 17, **dadurch gekennzeichnet, daß** die Vortrennung/Vorreinigung eine wäßrige Nukleinsäure-Zweiphasentrennung oder eine Anionenaustauscherchromatographie ist.
